Europäisches Patentamt

European Patent Office

Office européen des brevets

(11) Publication number: **0 261 892 B1**

# EUROPEAN PATENT SPECIFICATION

(45) Date of publication of patent specification: **02.01.92**

(51) Int. Cl.5: **C07C 51/265**, C07C 63/26, C07C 63/24, C07C 63/16, C07C 63/06, C07C 63/36, C07C 63/38

(21) Application number: **87308288.7**

(22) Date of filing: **18.09.87**

(54) **The production of aromatic carboxylic acids.**

(30) Priority: **26.09.86 JP 226131/86**

(43) Date of publication of application:
**30.03.88 Bulletin 88/13**

(45) Publication of the grant of the patent:
**02.01.92 Bulletin 92/01**

(84) Designated Contracting States:
**DE FR GB IT NL**

(56) References cited:
**EP-A- 0 135 341**
**BE-A- 903 542**
**GB-A- 825 429**
**GB-A- 1 264 815**
**GB-A- 2 106 797**

(73) Proprietor: **MITSUI PETROCHEMICAL INDUS-TRIES, LTD.**
**2-5, Kasumigaseki 3-chome Chiyoda-ku Tokyo 100(JP)**

(72) Inventor: **Shiraki, Shigemi**
**No. 11-18, Hirata 6-chome**
**Iwakuni-shi Yamaguchi(JP)**
Inventor: **Mizuno, Kenichi**
**No. 111-6, Minamiiwakunicho 2-chome**
**Iwakuni-shi Yamaguchi(JP)**

(74) Representative: **Perry, Robert Edward et al**
**GILL JENNINGS & EVERY 53-64 Chancery Lane**
**London WC2A 1HN(GB)**

## Description

### FIELD OF THE INVENTION

The present invention relates to a process for the production of aromatic carboxylic acids.

### PRIOR ART

There are two primary prior art processes for the production of aromatic carboxylic acids, e.g. terephthalic acid (TPA), by oxidising alkyl aromatic compounds such as xylenes in the liquid phase with an oxygen-containing gas in the presence of an oxidation catalyst. (1) BE-A-0903542 discloses separating the solvent vapour evaporated in the reactor from the exhausted reaction gas by condensing it and recirculating the thus-condensed solvent by returning it to the bottom of the reactor; (2) EP-A-0135341 discloses recirculating a predetermined proportion of the reaction gas delivered from the reactor by returning it to the reactor at a portion within the liquid phase region.

It has thus been recognised that reduced reaction temperature and reduced reaction pressure can be employed. Especially in the case (2), the oxidation reaction is effected with increasing oxygen partial pressure in the gas phase of the reaction system, whereby a reaction mixture containing TPA having improved light transmittance is obtained. It is reported that the hue of a subsequently-prepared polyester was superior even though the content of 4-carboxybenzaldehyde (4-CBA) amounted to more than 500 ppm, when the product was subjected to a post-oxidation treatment, and that an ultrapure product having a light transmittance ($T_{340}$) of some 95% could be obtained when the reaction was carried out under conditions such that the content of 4-CBA would have amounted to 200-300 ppm.

In the process (2), although the advantageous effect suggested above can be attained, there is considerable foaming on the liquid surface. In the continuous production of an aromatic carboxylic acid by oxidising an alkyl aromatic with oxygen-containing gas in the liquid phase and in the presence of a heavy metal compound and/or bromine-containing compound, the oxygen-containing gas is usually fed to the reactor in the liquid region, and the reaction is conducted under agitation, so that foaming occurs on the surface of liquid layer. The mist of reaction liquor containing the aromatic carboxylic acid suspended or dissolved therein, and originating from collapsed foam, will be entrained in the rising vapour and is carried over to the installation units such as heat exchanger and distillation tower. The result is problems in the succeeding process line, such as clogging of the line by the deposited carboxylic acid. Recirculation of the reaction gas to the liquid in the reactor aggravates the foaming problem. Further, any attempt to increase output and/or reduce costs by increasing the size of the reactor leads to an increase in the rate at which the vapour at the top of the reactor rises, and foaming is therefore a problem of greater significance.

Another important problem associated with recirculation of a part of the reaction gas freed from the condensing components by returning it to the liquid phase in the reactor is high power consumption by a gas recirculation blower. Further, the gas compression energy required for overcoming the hydrostatic head caused by the liquid layer is greater if large-scale apparatus is used.

### SUMMARY OF THE INVENTION

In a process according to the invention, a continuous process for the production of an aromatic carboxylic acid by oxidising an alkyl aromatic compound in the liquid phase with an oxygen-containing gas in a reactor, in the presence of a heavy metal compound and/or a bromine-containing compound, part of the reaction gas delivered from the reactor and freed from the condensing components is recirculated by returning it to the gas layer in the reactor.

### DESCRIPTION OF THE INVENTION

Alkyl aromatic compounds which can be used in the process according to the present invention include, for example, toluene, xylenes, trimethylbenzenes, methylnaphthalenes and dimethylnaphthalenes, beside other compounds such as p-tolualdehyde and p-toluic acid.

The aromatic carboxylic acid may be, for example, benzoic acid, phthalic acid, isophthalic acid, terephthalic acid, a benzenetricarboxylic acid, a naphthoic acid or a naphthalenedicarboxylic acid.

In a preferred embodiment of the invention, p-xylene is oxidised to terephthalic acid.

Since the process of the present invention involves liquid phase oxidation, the oxidation is carried out in a solvent. Examples of suitable reaction solvents are fatty acids such as acetic acid, propionic acid, butyric acid, isobutyric acid, pentanoic acid, pivalic acid and caproic acid, optionally in admixture with water. Acetic acid and acetic acid-water are preferred.

The catalyst employed in the process according to the present invention is a transition or heavy metal compound and/or a bromine-containing compound. Examples of suitable metals are nickel, cobalt, iron, chromium and manganese. Examples of suitable metal compounds include cobalt acetate, cobalt naphthenate, cobalt bromide, manganese acetate, manganese naphthenate, manganese bromide, nickel acetate, nickel bromide, iron acetate, iron bromide, chromium acetate and chromium bromide. Examples of suitable bromine-containing compounds include the bromides given above, hydrogen bromide, tetrabomoethane and aliphatic carboxylic acid bromoalkanol esters having 4-6 carbon atoms, such as dibromoethyl acetate and bromopropyl acetate.

Preferably, a cobalt compound and a manganese compound are employed concurrently with a bromine-containing compound. The amount of cobalt compound is usually 10 to 10,000, preferably 100 to 3,000, ppm, based on the weight of the solvent. The manganese:cobalt atomic ratio is usually 0.001:1 to 2:1. The bromine-containing compound:cobalt atomic ratio is usually 0.1:1 to 5:1.

The oyxgen-containing gas employed in the process according to the present invention is usually oxygen diluted by an inert gas, such as air or oxygen-enriched air.

The temperature of the oxidation reaction in the process according to the present invention is usually between 150 and 270$^\circ$C, preferably between 170 and 220$^\circ$C. The reaction pressure should be higher than that under which the liquid phase of the reaction mixture is maintained at the reaction temperature, and may, in general, be from 5 to 40 kg/cm$^2$ gauge. The reaction duration may be such that the residence time in the reactor is 20 to 180 minutes, but may depend on the specific size and the capacity of the reactor.

The reaction system generally contains water, e.g. in an amount of 3 to 30, preferably 5 to 15, % by weight. The water content can be adjusted by exhausting a suitable proportion of the condensate separated from the delivered reaction gas, that contains the condensed water, out of the reaction system by separating it from the portion of the condensate to be recirculated.

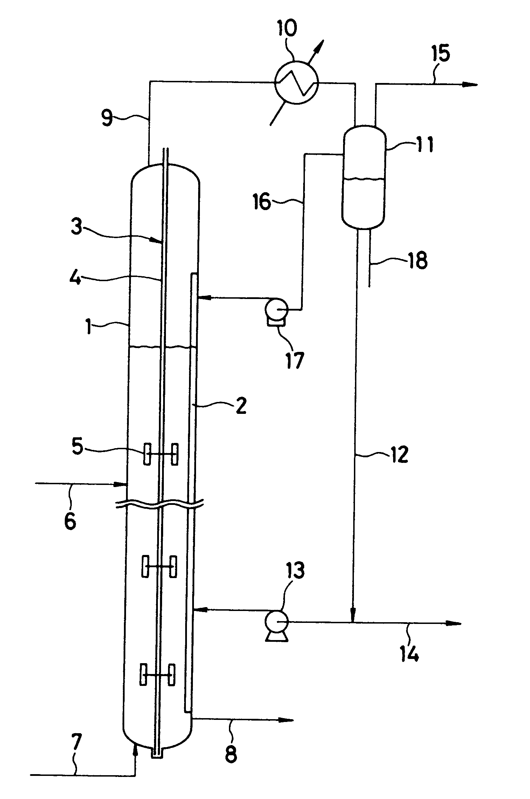

The invention will now be described by way of illustration only with reference to the accompanying drawing which is a schematic representation of apparatus which can be used in the novel process. The drawing shows a reactor 1 equipped with a baffle plate 2 near the inner surface of the reactor shell and, at its centre, with a stirrer 3 comprising a shaft 4 and a plurality of stir vanes 5. While the reactor 1 is usually of an agitation type, as shown in the drawing, the stirrer 3 may, on occasion, be dispended with.

The alkyl aromatic compound and the solvent are fed to the reactor 1 via a raw material feed line 6 and the oxygen-containing gas is supplied via a gas feed line 7. The reaction product is discharged from the reaction system via a product discharge line 8. At the top of the reactor 1, there is a reaction gas delivery line 9 in which there is a heat exchanger 10 for separating the condensing components from the delivered reaction gas. It is possible to install a distillation tower (not shown) instead of or together with the heat exchanger 10.

The condensing components condensed upon cooling by the heat exchanger 10 are separated from the gas components and are collected in a separator drum 11 as condensate. The separator 11 is provided with a gas recirculation line 16, a gas exhaustion line 15, a condensate outlet line 12 and a sampling line 18. A part of the condensate is recirculated within the system by returning it continuously to the reactor 1 by means of a pump 13; if necessary or desired, the remainder may be discharged. It is possible to recirculate all or a part of the condensate within the system by returning it to the region of the gas phase, instead of returning it to the region of the liquid layer in the reactor.

The gas separated from the condensing components is partly exhausted out of the system via the exhaustion line 15 and partly returned to the gas layer in the reactor via the gas circulation line 16 by means of a blower 17. As necessary or desired, the gas to be returned to the gas region of the reactor 1 is cooled by a cooler (not shown) to a temperature of, for example, 150$^\circ$C or below, preferably 120$^\circ$C or below. By returning the separated and cooled reaction gas to the gas layer of the reactor, the foam-suppression is enhanced.

The amount of the reaction gas recirculated to the reactor 1 is relative to the gas exhausted out of the reaction system (entire exhaustion gas) is usually 0.05:1 to 4:1, preferably 0.1:1 to 2:1, depending on the particular reactor and the conditions employed. The foam-suppressing effect is achieved within the given range and, therefore, the actual proportion should be chosen in this range, in order that reaction mixture vapour is not entrained by the recirculating gas itself. The point(s) at which the reaction gas to be recirculated is returned to the reactor 1 may be anywhere that a substantial foam-suppression effect can be attained. A level higher than one-tenth of the reactor diameter above the liquid surface may be preferred.

It is possible to bring about a swirling current in the gas region above the liquid surface of the reactor by providing a nozzle via which the recirculated gas is introduced into the reactor in an eccentric path; this may efficiently contribute to collapsing of foam on the liquid surface and, at the same time, blow the liquid droplets or mist floating in the gas space on to the reactor shell wall where they collect, in a manner similar to a cyclone separator. The nozzle may be arranged such that a substantially swirling stream is formed, e.g. at an angle of from 0 to 80$^\circ$ to the tangent.

The aromatic carboxylic acid formed by the process according to the present invention may be used as a commercial product after subjecting the reaction mixture to a conventional treatment process such as crystallisation or, for specific uses, to other known treatment procedures including crystallisation or high temperature post-oxidation and purification by hydrogenation.

By the process according the present invention, it is possible to increase the oxygen partial pressure in the gas phrase by recirculating part of the reaction gas; the result is an increase in the reaction pressure as compared to the case without recirculation of the reaction gas. Therefore, aromatic carboxylic acids such as terephthalic acid, exhibiting superior light transmittance, as in the case of BE-A-0903542, can be obtained with the further advantage of avoidance or minimisation of the foaming problem.

Another advantage of the present invention consists in the relative decrease in the combustion loss of the solvent, e.g. acetic acid, during the oxidation reaction. This depends on the fact that the reaction conditions can be relatively favourable in the process according to the present invention, for attaining a degree of oxidation comparable to a process without gas recirculation, since the oxygen partial pressure in the gas phase can be elevated. It is possible to realise such advantageous effects as lower power consumption for gas recirculation, by the reduction of compression pressure due to the exclusion of hydrostatic head. This is an important advantage over the process described in BE-A-0903542.

The following Example illustrates the invention. In the Example, the content of 4-CBA in the TPA product was determined by polarography; the light transmittance of the product was represented by light transmittance $T_{340}$ (%) at 340 m$\mu$ of a 2 N potassium hydroxide aqueous solution containing TPA at a concentration of 15% by weight.

Example

An oxidation reactor as shown in the drawing was employed. The reactor 1 was provided with a stirrer 3 composed of a rotary shaft 4 supported at two intermediate portions and at the bottom (not shown) and of stir vanes 5 disposed in five stages. The reactor 1 consisted of a vertical cylinder made of titanium having an inner diameter of 400 mm and a length (height) of 7 m.

The reactor was charged initially with 310 kg acetic acid, 22 kg water, 1637 g cobalt acetate, 8.1 g manganese acetate and 880 g tetrabromoethane. The mixture was maintained at a temperature of 186°C and a pressure of 12.8 kg/cm$^2$ gauge. The oxidation reaction was conducted continuously while feeding in 110 kg/hr p-xylene, 460 kg/hr acetic acid, 30 kg/hr water, 2460 g/hr cobalt acetate, 12.2 g/hr manganese acetate and 1320 g/hr tetrabromoethane (via the raw material feed line 6) and air (via the gas feed line 7) at such a rate that the oxygen concentration in the delivered reaction gas would have reached 6.4% by volume. The delivered reaction gas freed from the condensing components in the separator drum 11, in an amount that corresponds to 30% by volume of the exhaustion gas exhausted via the gas exhaustion line 15, was recirculated through the return line 16 by the blower 17 during the operation.

An injection nozzle for the recirculation gas was located 600 mm above the liquid level, in the gas region of the reactor 1. The gas introduced was directed at an angle of 30° to the tangent of the reactor shell towards the direction of rotation of the stirrer 3. The liquid reaction mixture was discharged out of the reactor via the discharge line 8 at a rate such that the residence time of the liquid reaction mixture within the reactor would have amounted to 40 minutes. The liquid reaction mixture thus discharged was subjected to solid/liquid separation and the product was washed with acetic acid. The TPA product exhibited a light transmittance $T_{340}$ of 73.7% and a 4-CBA content of 605 ppm. The power consumption by the gas circulation blower was as high as 3 kW.

The test run was continued for one week without interruption. A sample of liquid reaction mixture taken from the sampling line 18 was almost transparent, although slight turbidity was observed. On termination of the test run, the internal condition of the heat exchanger 10 was inspected by opening it; almost no deterioration over its internal surfaces was detected.

Comparative Example

The procedure of the Example was repeated, with the only exception that the reaction gas was returned to the reactor 1 via the line 16 at a position 500 mm from the bottom of the reactor, within the liquid layer. After 8 hours from the commencement of the operation, the test run was stopped due to the fact that the pressure drop between the inlet and the outlet of the heat exchanger 10 had reached an intolerable value. On inspecting the inside of the heat exchanger 10 by opening it, deposition of crystalline TPA was found on the inlet side, and the throughput in the cooling pipe row of the heat exchanger had been reduced to about 20%. The product thus obtained exhibited a light transmittance of 70.5% and a 4-CBA content of 666 ppm, which were nearly the same as the values in the Example of the invention; the power consumption of the gas circulation was 7 kW and, at the end of the test run, had increased to about 9 kW.

## Claims

1. A process for the continuous production of an aromatic carboxylic acid by oxidising an alkyl aromatic compound in the liquid phase with an oxygen-containing gas in a reactor, in the presence of a metal compound and/or a bromine-containing compound, which comprises recirculating a part of the reaction gas delivered from the reactor and freed from the condensing components by returning it to the gas region in the reactor.

2. A process according to claim 1, wherein a part of the condensing components is recirculated by returning it to the liquid region in the reactor.

3. A process according to claim 1 or claim 2, wherein the alkyl aromatic compound is selected from toluene, xylenes, trimethylbenzenes, methylnaphthalenes, dimethylnaphthalenes, p-tolualdehyde and p-toluic acid.

4. A process according to any preceding claim, wherein the aromatic carboxylic acid is selected from benzoic acid, phthalic acid, isophthalic acid, terephthalic acid, benzenetricarboxylic acid, naphthoic acids and naphthalenedicarboxylic acids.

## Revendications

1. Procédé pour la production continue d'un acide carboxylique aromatique par oxydation d'un composé alkylaromatique en phase liquide avec un gaz contenant de l'oxygène dans un réacteur, en présence d'un composé métallique et/ou d'un composé bromé, oui comprend le recyclage d'une partie du gaz réactionnel, sortant du réacteur et débarrassée des composants qui se condensent, par renvoi dans la région de gaz du réacteur.

2. Procédé selon la revendication 1, dans lequel une partie des composants qui se condensent est recyclée par renvoi dans la région de liquide du réacteur.

3. Procédé selon la revendication 1 ou la revendication 2, dans lequel le composé alkylaromatique est choisi parmi le toluène, les xylènes, les triméthylbenzènes, les méthylnaphtalènes, les diméthylnaphtalènes, le p-tolualdéhyde et l'acide p-toluique.

4. Procédé selon l'une quelconque des revendications précédentes, dans lequel l'acide carboxylique aromatique est choisi parmi l'acide benzoïque, l'acide phtalique, l'acide isophtalique, l'acide téréphtalique, les acides benzènetricarboxyliques, l'acide naphtoïque et les acides naphtalènedicarboxyliques.

## Patentansprüche

1. Verfahren zur kontinuierlichen Herstellung einer aromatischen Carbonsäure durch Oxidieren einer alkylaromatischen Verbindung in flüssiger Phase mit einem Sauerstoff-haltigen Gas in einem Reaktor in Anwesenheit einer Metallverbindung und/oder einer Brom enthaltenden Verbindung, umfassend die Rückführung eines Teils des Reaktionsgases, das vom Reaktor abgeführt und von kondensierenden Bestandteilen befreit wurde, durch Zurückleiten in die Gasregion im Reaktor.

2. Verfahren nach Anspruch 1, worin ein Teil der kondensierenden Bestandteile rückgeführt wird, indem er in die flüssige Region im Reaktor zurückgeleitet wird.

3. Verfahren nach Anspruch 1 oder 2, worin die alkylaromatische Verbindung aus Toluol, Xylolen, Trimethylbenzolen, Methylnaphthalinen, Dimethylnaphthalinen, p-Toluylaldehyd und p-Toluylsäure ausgewählt ist.

4. Verfahren nach irgendeinem der vorhergehenden Ansprüche, worin die aromatische Carbonsäure aus Benzoesäure, Phthalsäure, Isophthalsäure, Terephthalsäure, Benzoltricarbonsäure, Naphthoesäuren und Naphthalindicarbonsäuren ausgewählt ist.